# EUROPÄISCHE PATENTSCHRIFT

(11) **EP 1 212 273 B1**
(45) Veröffentlichungstag und Bekanntmachung des Hinweises auf die Patenterteilung: **02.02.2005**
(21) Anmeldenummer: 00972657.1
(22) Anmeldetag: 31.08.2000
(51) Int. Cl.: C07C 51/00

(54) **VERFAHREN ZUR REINIGUNG VON CARBONSÄURECHLORIDEN**
METHOD FOR PURIFYING ACID CHLORIDES
PROCEDE DE PURIFICATION DE CHLORURES D'ACIDE CARBONIQUE

(30) Priorität: 13.09.1999 DE 19943858
(43) Veröffentlichungstag der Anmeldung: 12.06.2002
(73) Patentinhaber: BASF AKTIENGESELLSCHAFT, 67056 Ludwigshafen (DE)
(72) Erfinder: BUSCH, Ralph, 67549 Worms (DE); KNEUPER, Heinz-Josef, 67150 Niederkirchen (DE); WEBER, Theodor, 67063 Ludwigshafen (DE); MÜLLER, Winfried, 68163 Mannheim (DE); STAMM, Armin, 55268 Nieder-Olm (DE); HENKELMANN, Jochem, 68165 Mannheim (DE)
(86) Internationale Anmeldenummer: PCT/EP2000/008514
(87) Internationale Veröffentlichungsnummer: WO 2001/019767

(56) Entgegenhaltungen:
- DE-A- 19 943 844
- US-A- 4 900 479
- US-A- 5 166 427

## Beschreibung

Die vorliegende Erfindung betrifft ein Verfahren zur Reinigung von Carbonsäurechloriden, welche aus der Umsetzung der entsprechenden Carbonsäuren mit Phosgen oder Thionylchlorid stammen und welches zu Carbonsäurechloriden mit verbesserter Farbzahl führt.

Carbonsäurechloride sind wichtige Zwischenprodukte bei der Synthese einer Vielzahl chemischer Produkte, insbesondere Pharmazeutika, Kosmetika, Tenside und Papierhilfsmittel. Sie können durch Umsetzung von Carbonsäuren mit Chlorierungsmitteln, wie PCl₃, POCl₃, SOCl₂, SO₂Cl₂ oder COCl₂ hergestellt werden. Von technischer Bedeutung sind vor allem die Umsetzungen mit Thionylchlorid, Phosphortrichlorid und Phosgen.

Bei der Synthese über Phosphortrichlorid wird im allgemeinen ein Reaktand (Carbonsäure oder Phosphortrichlorid) vorgelegt und der andere Reaktand (Phosphortrichlorid oder Carbonsäure) langsam zugeführt. Gegebenenfalls wird die Synthese in einer, mit einem reaktionsinerten Lösungsmittel (z.B. Toluol) verdünnten Lösung durchgeführt. Nach Abtrennung der gebildeten phosphorigen Säure erfolgt in der Regel eine destillative Reinigung des Carbonsäurechlorids. Der Zusatz eines Katalysators ist nicht erforderlich.

EP-A-0 296 404 beschreibt die Reinigung von rohen Carbonsäurechloriden, welche aus der Chlorierung mittels Phosphortrichlorid stammen, bei der die Reaktionsprodukte mit Carbonsäureamid-hydrohalogeniden behandelt werden. Die Carbonsäurechlorid-Rohlösungen der Phosphortrichlorid-Route unterscheiden sich in der Zusammensetzung von denen der Phosgen- bzw. Thionylchloridroute erheblich. So weisen letztere auf:
(i) Einen wesentlich höheren Gehalt an störenden Nebenkomponenten.
(ii) Eine unterschiedliche Zusammensetzung der Nebenkomponenten, welche durch die Wahl des Chlorierungs-Agens beeinflußt wird.
(iii)Ergänzend zur unterschiedlichen Zusammensetzung der Nebenkomponenten, noch die Anwesenheit von Abbau- und/oder Folgeprodukten aus den eingesetzten Katalysator-Addukten.

US 5,166,427 beschreibt ein Verfahren zur Herstellung von Carbonsäurechloriden durch Umsetzung von Carbonsäuren mit Phosgen in Gegenwart eines Katalysator-Addukts aus einem N,N-disubstituierten Formamid und Phosgen, bei dem man das Gemisch nach der Umsetzung in zwei Phasen separieren lässt, anschließend trennt und die Katalysator-Addukt haltige Phase wiederverwendet.

Die Verwendung von Phosgen oder Thionylchlorid anstelle von Phosphortrichlorid führt in der Regel zu einem höheren Umsatz und einer besseren Selektivität. Beide Chlorierungsmittel haben gegenüber Phosphortrichlorid zudem den Vorteil, daß nur gasförmige Nebenprodukte gebildet werden, die entweder während der Synthese gasförmig entweichen oder durch Strippung mit einem Inertgas nach beendeter Reaktion vollständig ausgetrieben werden können. Desweiteren ist speziell Phosgen ein sehr preiswertes Chlorierungsmittel.

Im Gegensatz zu Phosphortrichlorid als Chlorierungsmittel sind Thionylchlorid und vor allem Phosgen weniger reaktiv. Die Herstellung von Carbonsäurechloriden durch Umsetzung von Carbonsäuren mit Thionylchlorid wird daher zur Erhöhung der Reaktionsgeschwindigkeit vorzugsweise in Gegenwart eines Katalysators durchgeführt. Bei der Herstellung durch Umsetzung mit Phosgen wird stets ein Katalysator eingesetzt. Für beide Chlorierungsmittel geeignete Katalysatorvorstufen sind N,N-disubstituierte Formamide und deren Hydrochloride, aber auch Pyridin oder Harnstoff. Übersichten, betreffend die Chlorierung mittels Thionylchlorid, sind gegeben in M.F. Ansell in S. Patai, "The Chemistry of Acyl Halides", John Wiley and Sons, New York 1972, 35-69 und H.H. Bosshard et al., Helv. Chem. Acta 62 (1959) 1653-1658 sowie S.S. Pizey, Synthetic Reagents, Vol. 1, John Wiley and Sons, New York 1974, ISBN 853120056, 321-557, speziell 333-335. Sowohl nach der Phosgen-Route als auch nach der Thionylchlorid-Route werden N,N-disubstituierte Formamide bevorzugt eingesetzt. Diese setzen sich mit den genannten Chlorierungsmitteln zu den sogenannten Vilsmeier-Salzen um.

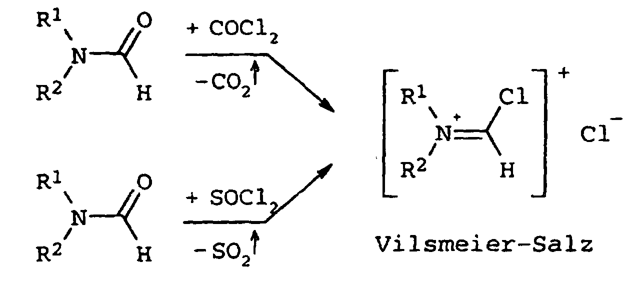

Das Vilsmeier-Salz, das eigentlich reaktive Chlorierungsreagenz, reagiert mit der Carbonsäure oder dem Carbonsäureanhydrid zum Säurechlorid. Dabei wird Formamid-Hydrochlorid zurückgebildet, das wiederum mit Phosgen oder Thionylchlorid zum Vilsmeier-Salz reagieren kann und weitere Katalysatorkreisläufe durchläuft. Die N,N-disubstituierten Formamid-Hydrochloride bzw. deren Vilsmeier-Salze sind jedoch thermisch nicht sehr stabil, so daß es oberhalb von 80 bis 90°C zu Nebenreaktionen kommen kann.

Die bevorzugte Verwendung von N,N-disubstituierten Formamiden als Katalysatorvorstufe für die Phosgenierung von Carbonsäuren geht auch aus EP-A-0 367 050, EP-A-0 452 806, DE-A-4 337 785, EP-A-0 475 137 und EP-A-0 635 473 hervor.

In Bezug auf die Farbzahl wirkt sich bei der Chlorierung von Carbonsäuren mit Phosgen oder Thionylchlorid der Einsatz von Katalysatoren nachteilig aus. Diese werden zwar nach der Chlorierung durch Phasentrennung abgetrennt, können aber in geringen Mengen im Produkt verbleiben und entweder selbst oder als Abbauoder Folgeprodukte zu Gelbfärbungen der Carbonsäurechloride führen. Im allgemeinen werden daher die über Phosgen oder Thionylchlorid hergestellten Carbonsäurechloride destillativ zu weitgehend farblosen Produkten gereinigt. Eine solche Destillation ist nicht nur ein energie- und zeitaufwendiger Vorgang, sondern birgt auch noch eine Reihe weiterer Nachteile. Viele längerkettige Carbonsäurechloride lassen sich nicht ohne partielle Zersetzung destillieren. Weiter ist bekannt, daß durch Zersetzung des im Destillationssumpf noch vorhandenen Katalysators die destillierten Produkte verunreinigt werden können. Größere Mengen an aufgepegeltem Katalysatorrückstand stellen bei der Destillation auch ein Sicherheitsrisiko dar, da in der Hitze die Gefahr einer spontanen Zersetzung besteht.

Eine weitere Möglichkeit zur Reinigung der rohen Carbonsäurechloride ist die Behandlung mit Aktivkohlen. Diese absorptiven Reinigungsschritte sind jedoch technisch aufwendig und zudem nicht immer erfolgreich. Des weiteren fällt kontaminierter Feststoff an, welcher anschließend fachgerecht entsorgt werden muß.

Es bestand daher die Aufgabe, ein Verfahren zur Reinigung von Carbonsäurechloriden, welche zum überwiegenden Teil aus der Umsetzung von Carbonsäuren mit Phosgen oder Thionylchlorid stammen, zu entwickeln, welches die bekannten Nachteile nicht mehr besitzt und zu Carbonsäurechloriden mit verbesserter Farbzahl führt.

Die Aufgabe wurde gelöst durch die Entwicklung eines verfahrens zur Reinigung von Carbonsäurechloriden, die durch Umsetzung von Carbonsäuren mit Phosgen oder Thionylchlorid in Gegenwart eines Katalysator-Addukts hergestellt und von der das Katalysator-Addukt erhaltenden Phase abgetrennt wurden, das dadurch gekennzeichnet ist, daß man die Carbonsäurechloride mit einem Hydrohalogenid von Carbonsäureamiden der allgemeinen Formel (I) 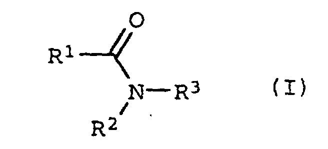 in der R¹ für Wasserstoff oder C₁- bis C₃-Alkyl steht; R² und R³ unabhängig voneinander C₁- bis C₄-Alkyl oder R² und R³ gemeinsam eine C₄- oder C₅-Alkylenkette bedeuten, behandelt und das so gereinigte Carbonsäurechlorid durch Abtrennung von der Carbonsäureamid-hydrohalogenid-Phase isoliert.

Nach dem erfindungsgemäßen Verfahren lassen sich verunreinigte Carbonsäurechloride, welche aus der Umsetzung von Carbonsäuren mit Phosgen oder Thionylchlorid stammen, in hoher Ausbeute und mit verbesserter Farbzahl extraktiv aufarbeiten. Unter verbesserter Farbzahl ist dabei bei erstmaliger Behandlung der Rohlösungen bei gesättigten Carbonsäurechloriden eine Verringerung der Farbzahl nach APHA auf weniger als 50% des ursprünglichen Wertes und bei ungesättigten Carbonsäurechloriden eine Verringerung der Iodfarbzahl auf weniger als 75% des ursprünglichen Wertes zu verstehen. Die Bestimmungen der Farbzahl nach APHA und der Iodfarbzahl werden in der Norm DIN EN 1557 (März 1997) beschrieben.

Die Behandlung der Carbonsäurechlorid-Rohlösung kann sowohl räumlich als auch zeitlich von der Synthese der Rohlösung getrennt sein. Die Behandlung mit einem Hydrohalogenid von Carbonsäureamiden der allgemeinen Formel (I) kann also auch in einem anderen Apparat als die Synthese des Carbonsäurechlorides durchgeführt werden. Synthese und Behandlung der Carbonsäurechlorid-Rohlösung können zwar zeitlich direkt aufeinander folgen, es besteht aber auch die Möglichkeit einer zeitlichen Trennung von Stunden, Tagen, Monaten oder Jahren, so daß auch eine Zwischenlagerung oder ein Transport der Rohlösung eingeschlossen ist.

Zur Behandlung der Carbonsäurechlorid-Rohlösung nach dem erfindungsgemäßen Verfahren wird diese in einem Apparat, welcher auch mit dem zuvor genutzten Reaktionsapparat identisch sein kann, mit einem Hydrohalogenid von Carbonsäureamiden der allgemeinen Formel (I) 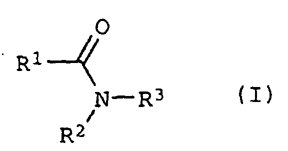 in der die Substituenten folgende Bedeutung haben:
- R¹: Wasserstoff oder C₁- bis C₃-Alkyl, konkret Methyl, Ethyl, Propyl oder 1-Methylethyl; besonders bevorzugt Wasserstoff;
- R² und R³: unabhängig voneinander ein C₁- bis C₄-Alkyl, konkret Methyl, Ethyl, Propyl, 1-Methylethyl, Butyl, 1-Methylpropyl, 2-Methylpropyl oder 1,1-Dimethylethyl, oder gemeinsam eine C₄- oder C₅-Alkylenkette, konkret CH₂CH₂CH₂CH₂ oder CH₂CH₂CH₂CH₂CH₂; besonders bevorzugt Methyl,
versetzt.

Wesentlich ist, daß die gegenseitige Löslichkeit der Carbonsäurechloride und der Hydrohalogenide der Carbonsäureamide (I) gering ist und sich zwei isolierbare Phasen ausbilden.

Die Menge der zuzugebenden Hydrohalogenide der Carbonsäureamide (I) ist von verschiedenen Faktoren, vor allem jedoch durch die Art des Carbonsäurechlorids selbst und die Menge an vorhandenen Nebenkomponenten in der Carbonsäurechlorid-Rohlösung, welche sich wiederum durch die Farbgebung bemerkbar machen abhängig. Bezogen auf die Menge des Carbonsäurechlorids sind im allgemeinen 1 bis 80 Gew.-%, bevorzugt 2 bis 60 Gew.-% und besonders bevorzugt 5 bis 50 Gew.-% an Hydrohalogenid der Carbonsäureamide (I) einzusetzen.

Als Hydrohalogenid der Carbonsäureamide (I) wird bevorzugt das Hydrochlorid, besonders bevorzugt das Hydrochlorid von N,N-Dimethylformamid eingesetzt. Der molare Anteil von Hydrochlorid (als HCl), bezogen auf das N,N-Dimethylformamid, liegt im Bereich zwischen 0,1 und 2,5. Bevorzugt eingesetzt wird ein molarer Anteil von 1,0 bis 2,0.

Die Herstellung der Carbonsäureamid-hydrohalogenide aus Carbonsäureamiden (I) und Halogenwasserstoff kann sowohl vor Zugabe des Carbonsäurechlorids als auch nach dessen Zugabe erfolgen.

Die Behandlung der Carbonsäurechlorid-Rohlösung mit den Hydrohalogeniden der Carbonsäureamide (I) erfolgt vorzugsweise bei einer Temperatur von -15 bis 80°C, bevorzugt -10 bis 40°C, besonders bevorzugt bei 0 bis 30°C und einem Druck von 0,5 bis 5 bar abs, bevorzugt 0,8 bis 1,2 bar abs unter intensiver Durchmischung. Die einzustellende Parameter richten sich dabei nach dem gewünschten Restgehalt an Carbonsäureamid-hydrohalogenid in der Carbonsäurechlorid-Phase und ist für jedes System mit den für den Fachmann bekannten vorgehensweisen anzupassen. Die Zeitdauer richtet sich im wesentlichen nach der Löslichkeit der unerwünschten Nebenkomponenten in der Carbonsäureamid-hydrohalogenid-Phase und ist ebenfalls am jeweiligen System zu bestimmen. Im allgemeinen wird die intensive Durchmischung für maximal 1 Stunde durchgeführt.

Die Behandlung kann sowohl diskontinuierlich als auch kontinuierlich durchgeführt werden.
(a) diskontinuierliche Behandlung:
   Bei der diskontinuierlichen Behandlung gibt man die Carbonsäurechlorid-Rohlösung und die Carbonsäureamid-hydrohalogenid-Phase in einem Apparat zusammen und durchmischt das System intensiv wie oben beschrieben. Geeignete Apparate sind beispielsweise Rührkessel oder Phasentrenngefäße ("Mixer-Settler"). Nach Beendigung der Durchmischung erfolgt die Trennung der beiden Phasen. Diese kann nun im bereits eingesetzten Behandlungs- oder Mischungsapparat erfolgen oder in. einem weiteren Apparat, beispielsweise einem Trenngefäß. Im allgemeinen haben sich beide Phasen nach maximal 2 Stunden getrennt und können nun isoliert werden.
(b) kontinuierliche Behandlung:
   Bei der kontinuierlichen Behandlung führt man die Carbonsäurechlorid-Rohlösung und die Carbonsäureamid-hydrohalogenid-Phase kontinuierlich einem Behandlungs- oder Mischungsapparat zu. Die Behandlung kann in bekannter Weise in Rührkesseln, Rührkesselkaskaden, statischen Mischern, Phasentrenngefäßen ("Mixer-Settler") oder flüssig-flüssig-Extraktionskolonnen (siehe Ullmann's Encyclopedia of Industrial Chemistry, 6^{th} edition, 1998, Electronic Release, Liquid-liquid-extraction) erfolgen. Eine der zugeführten Menge beider Phasen entsprechende Menge wird kontinuierlich aus dem Behandlungsoder Mischungsapparat abgeführt. Dabei ist darauf zu achten, daß das Verhältnis zwischen Carbonsäurechlorid und Carbonsäureamid-hydrohalogenid nahezu konstant bleibt. Die entnommene Menge wird einem weiteren Apparat, beispielsweise einem Trenngefäß zur Trennung der beiden Phasen, zugeführt. Dabei kann auch eine Beruhigungszone zwischen Behandlungs- oder Mischungsapparat und Trenngefäß dazwischengeschaltet sein. Dem Trenngefäß können beide Phasen separat entnommen werden.

Zur Abtrennung der Carbonsäureamid-hydrohalogenid-haltigen Phase können auch geeignete Filter, wie beispielsweise Koaleszierfilter bekannter Bauart, eingesetzt werden. Die abgetrennte Carbonsäureämid-hydrohalogenid-haltige Phase kann gegebenenfalls erneut zur Extraktion eingesetzt werden, wobei die in die Carbonsäurechlorid-Phase übergegangene Menge an Halogenwasserstoff vorteilhaft zu ersetzen ist.

Die derart behandelten Carbonsäurechlorid-Lösungen zeigen gegenüber den unbehandelten eine verbesserte Farbzahl und können nun entweder direkt für weitere Synthesestufen eingesetzt werden oder bei Bedarf noch weiteren Behandlungsprozeduren unterzogen werden. Hierzu seien ohne Limitierung genannt die erneute Behandlung mit einem Hydrohalogenid von Carbonsäureamiden (I) nach dem erfindungsgemäßen Verfahren, die Destillation oder die Adsorptivreinigung.

In einer weiteren Ausführungsform wird die abgetrennte Carbonsäureamid-hydrohalogenid-Phase anschließend als Katalysatorvorstufe zur Bildung des Katalysator-Addukts aus Phosgen bzw. Thionylchlorid und dem N,N-disubstituierten Formamid eingesetzt. Hierzu behandelt man die abgetrennte Carbonsäureamid-hydrohalogenid-Phase mit Phosgen bzw. Thionylchlorid, um es anschließend als Katalysator-Addukt einzusetzen.

Nach dem erfindungsgemäßen Verfahren herstellbare Carbonsäurechloride sind beispielsweise solche der allgemeinen Formel (III) 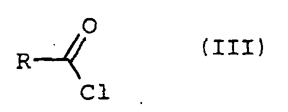 in der R für folgende Reste steht:
C₁- bis C₃₀-Alkyl oder deren aryl- oder cycloalkyl-substituierte Komponenten:
   gesättigter, geradkettiger oder verzweigter Kohlenwasserstoffrest mit 1 bis 30 C-Atomen, bevorzugt Methyl, Ethyl, Propyl, 1-Methylethyl, Butyl, 1-Methylpropyl, 2-Methylpropyl, 1,1-Dimethylethyl, Pentyl, 1-Ethylpropyl, Hexyl, Heptyl, 1-Ethylpentyl, Octyl, 2,4,4-Trimethylpentyl, Nonyl, 1,1-Dimethylheptyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosy, Octacosyl, Nonacosyl, Triacontyl, Phenylmethyl, Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
C₃-bis C₁₂-Cycloalkyl oder deren aryl- oder cycloalkyl-substituierte Komponenten:
   monocyclischer, gesättigter Kohlenwasserstoffrest mit 3 bis 12 Ring-C-Atomen, bevorzugt Cyclopentyl, Cyclohexyl;
C₂-bis C₃₀-Alkenyl oder deren aryl- oder cycloalkyl-substituierte Komponenten:
   ungesättigter, geradkettiger oder verzweigter Kohlenwasserstoffrest mit 1 bis 30 C-Atomen und 1 bis 5 Doppelbindungen an einer beliebigen Stelle, bevorzugt 2-Propenyl, 3-Butenyl, cis-2-Butenyl, trans-2-Butenyl, cis-8-Heptadecenyl, trans-8-Heptadecenyl, cis,cis-8,11-Heptadecadienyl, cis,cis,cis-8,11,14-Heptadecatrienyl;
C₃-bis C₁₂-Cycloalkenyl oder deren aryl- oder cycloalkyl-substituierte Komponenten:
   monocyclischer, ungesättigter Kohlenwasserstoffrest mit 3 bis 12 Ring-C-Atomen und 1 bis 3 Doppelbindungen an einer beliebigen Stelle, bevorzugt 3-Cyclopentenyl, 2-Cyclohexenyl, 3-Cyclohexenyl, 2,5-Cyclohexadienyl;
C₂-bis C₃₀-Alkinyl oder deren aryl- oder cycloalkyl-substituierte Komponenten:
   ungesättigter, geradkettiger oder verzweigter Kohlenwasserstoffrest mit 1 bis 30 C-Atomen und 1 bis 3 Dreifachbindungen an einer beliebigen Stelle, bevorzugt 3-Butinyl, 4-Pentinyl;
C₄-bis C₃₀-Alkeninyl oder deren aryl- oder cycloalkyl-substituierte Komponenten:
   ungesättigter, geradkettiger oder verzweigter Kohlenwasserstoffrest mit 1 bis 30 C-Atomen, 1 bis 3 Dreifachbindungen und 1 bis 3 Doppelbindungen an einer beliebigen Stelle.

Mit dem erfindungsgemäßen Verfahren können auch Mischungen der genannten Carbonsäurechloride gereinigt werden. Als nicht-limitierende Beispiele seien genannt Mischungen aus C₈- bis C₁₈-Carbonsäurechloriden, welche unter den Trivialnamen "Carbonsäurechlorid", "Talgfettsäurechlorid", "Kokosfettsäurechlorid" und "Ölsäurechlorid" gehandelt werden.

Besonders bevorzugt hergestellt werden nach dem erfindungsgemäßen Verfahren Carbonsäurechloride der allgemeinen Formel (III), in der R für folgende Reste steht:
C₁-bis C₃₀-Alkyl oder deren aryl- oder cycloalkyl-substituierte Komponenten:
   gesättigter, geradkettiger oder verzweigter Kohlenwasserstoffrest mit 1 bis 30 C-Atomen, bevorzugt Methyl, Ethyl, Propyl, 1-Methylethyl, Butyl, 1-Methylpropyl, 2-Methylpropyl, 1,1-Dimethylethyl, Pentyl, 1-Ethylpropyl, Hexyl, Heptyl, 1-Ethylpentyl, Octyl, 2,4,4-Trimethylpentyl, Nonyl, 1,1-Dimethylheptyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenylmethyl, Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl;
C₂-bis C₃₀-Alkenyl oder deren aryl- oder cycloalkyl-substituierte Komponenten:
   ungesättigter, geradkettiger oder verzweigter Kohlenwasserstoffrest mit 1 bis 30 C-Atomen und 1 bis 5 Doppelbindungen an einer beliebigen Stelle, bevorzugt 2-Propenyl, 3-Butenyl, cis-2-Butenyl, trans-2-Butenyl, cis-8-Heptadecenyl, trans-8-Heptadecenyl, cis,cis-8,11-Heptadecadienyl, cis,cis,cis-8,11,14-Heptadecatrienyl;
sowie deren Mischungen.

Ganz besonders bevorzugt hergestellt werden nach dem erfindungsgemäßen Verfahren Essigsäurechlorid (R gleich Methyl), Propionsäurechlorid (R gleich Ethyl), Buttersäurechlorid (R gleich Propyl), Valeriansäurechlorid (R gleich Butyl), Isovaleriansäurechlorid (R gleich 2-Methylpropyl), Pivalinsäurechlorid (R gleich 1,1-Dimethylethyl), Capronsäurechlorid (R gleich Pentyl), 2-Ethylbuttersäurechlorid (R gleich 1-Ethylpropyl), Önanthsäurechlorid (R gleich Hexyl), Caprylsäurechlorid (R gleich Heptyl), 2-Ethylhexansäurechlorid (R gleich 1-Ethylpentyl), Pelargonsäurechlorid (R gleich Octyl), Isononansäurechlorid (R gleich 2,4,4-Trimethylpentyl), Caprinsäurechlorid (R gleich Nonyl), Neodecansäurechlorid (R gleich 1,1-Dimethylheptyl), Laurinsäurechlorid (R gleich Undecyl), Myristinsäurechlorid (R gleich Tridecyl), Palmitinsäurechlorid (R gleich Pentadecyl), Stearinsäurechlorid (R gleich Heptadecyl), Ölsäurechlorid (R gleich cis-8-Heptadecenyl), Linolsäurechlorid (R gleich cis,cis-8,11-Heptadecadienyl), Linolensäurechlorid (R gleich cis,cis,cis-8,11,14-Heptadecatrienyl), Arachidinsäurechlorid (R gleich Nonadecyl) und Behensäurechlorid (R gleich Henicosyl) sowie deren Mischungen.

Die für das erfindungsgemäße Verfahren vorteilhaft einzusetzenden Carbonsäuren gemäß Formel (III) ergeben sich aus den oben beschriebenen Definitionen für R.

Bei der Herstellung der Carbonsäurechlorid-Rohlösung wird als Katalysator ein sogenanntes Katalysator-Addukt eingesetzt, welches aus der Umsetzung von Phosgen oder Thionylchlorid mit einem N,N-disubstituierten Formamid stammt. Letzteres, welches auch als Katalysatorvorstufe zu bezeichnen ist, ist bestimmt durch die allgemeine Formel (II) 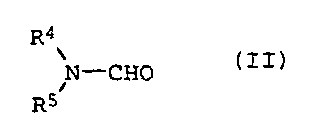 in der R⁴ und R⁵ unabhängig voneinander ein C₁- bis C₄-Alkyl, konkret Methyl, Ethyl, Propyl, 1-Methylethyl, Butyl, 1-Methylpropyl, 2-Methylpropyl und 1,1-Dimethylethyl, oder gemeinsam eine C₄- oder C₅-Alkylenkette, konkret CH₂CH₂CH₂CH₂ oder CH₂CH₂CH₂CH₂CH₂, bedeuten. Bevorzugt eingesetzt wird N,N-Dimethylformamid.

Die Herstellung der im erfindungsgemäßen Verfahren einzusetzenden Carbonsäurechloride aus der Umsetzung von Carbonsäuren mit Phosgen oder Thionylchlorid erfolgt nach allgemein bekannten verfahren nach dem Stand der Technik.

Die Bildung des Katalysator-Addukts kann sowohl in dem Apparat erfolgen, in dem die Chlorierung durchgeführt wird, als auch vorgelagert in einem anderen Apparat. Im letztgenannten Fall wird eine bestimmte Menge des N,N-disubstituierten Formamids in einem separaten Apparat vorgelegt, mit der gewünschten Menge an Phosgen oder Thionylchlorid beaufschlagt und anschließend dem eigentlichen Reaktionsapparat zugeführt. Im erstgenannten Fall wird die beschriebene Prozedur direkt im Reaktionsapparat durchgeführt.

Die Umsetzung der Carbonsäuren mit Phosgen oder Thionylchlorid in Gegenwart des beschriebenen Katalysator-Addukts kann sowohl diskontinuierlich, als auch kontinuierlich erfolgen. In der Phosgenvariante ist ein Molverhältnis zwischen dem Katalysator-Addukt und der Carbonsäure von 0,05 bis 2,0, bevorzugt 0,1 bis 1,0, besonders bevorzugt 0,1 bis 0,3 einzustellen, in der Thionylchlorid-variante ein Molverhältnis von 0,001 bis 0,05, bevorzugt 0,001 bis 0,01. In beiden Varianten wird die Umsetzung durchgeführt bei Temperaturen zwischen 20 und 100°C, bevorzugt zwischen 30 und 80°C, besonders bevorzugt zwischen 30 und 70°C und einem Druck zwischen 0,5 und 2,0 bar abs, bevorzugt 0,8 bis 1,2 bar abs, besonders bevorzugt bei Atmosphärendruck. Die insgesamt zugegebene Menge an Phosgen oder Thionylchlorid beträgt 1,0 bis 2,0 der Molmenge der eingesetzten Carbonsäure, bevorzugt 1,0 bis 1,3 der Molmenge der eingesetzten Carbonsäure.
(a) Diskontinuierliche Herstellung:
   Bei der diskontinuierlichen Herstellung wird das Reaktionsgemisch, bestehend aus einer Carbonsäure und dem Katalysator-Addukt, hergestellt aus Phosgen bzw. Thionylchlorid und dem N,N-disubstituierten Formamid der Formel (II), in einem Reaktionsapparat, beispielsweise einem Rührkessel, vorgelegt und auf Reaktionstemperatur und gegebenenfalls auf Reaktionsdruck gebracht. Nun wird die gewünschte Menge an flüssigem oder gasförmigem Phosgen oder Thionylchlorid über einen bestimmten Zeitraum zugegeben. Der Zeitbedarf für die Zugabe des Chlorierungsmittels richtet sich nach der Reaktionsgeschwindigkeit und kann im allgemeinen auf wenige Stunden begrenzt werden. Anschließend läßt man die Reaktionslösung im allgemeinen 1 bis 2 Stunden absitzen und trennt die beiden Phasen voneinander. In der Regel befindet sich die Carbonsäurechlorid-enthaltende Phase oben, die Katalysator-Addukt-enthaltende unten.
(b) Kontinuierliche Herstellung:
   Für die kontinuierliche Fahrweise geeignete Reaktionsapparate sind beispielsweise Rührkessel, Rührkesselkaskaden oder im Gegenstrom betriebene Reaktionskolonnen. Bei Verwendung eines Rührkessels legt man die Carbonsäure und das Katalysator-Addukt, hergestellt aus Phosgen bzw. Thionylchlorid und dem N,N-disubstituierten Formamid der Formel (II), vor, stellt die gewünschte Reaktionstemperatur und gegebenenfalls den gewünschten Reaktionsdruck ein und gibt flüssiges oder gasförmiges Phosgen oder Thionylchlorid zu. Nach Einleiten einer der Carbonsäure etwa äquivalenten Menge an Chlorierungsmittel beginnt man gleichzeitig Carbonsäure und das Katalysator-Addukt sowie eine, im wesentlichen der zugeführten Carbonsäure äquimolaren Menge an Phosgen bzw. Thionylchlorid, zuzufahren. Eine der zugefahrenen Reaktanden entsprechende Menge des Reaktionsvolumens wird dem Reaktionsapparat, beispielsweise über eine Standhaltung, entnommen und in ein Trenngefäß geleitet. Im Trenngefäß kann das Carbonsäurechlorid der Formel (III) als obere Phase kontinuierlich entnommen und das Katalysator-Addukt als untere Phase kontinuierlich dem Reaktor zurückgeführt werden. Bei der Reaktionsführung ist darauf zu achten, daß das durch die Reaktionsabgase mitgerissene Chlorierungsmittel durch zusätzlich zugeführtes ausgeglichen wird.

Die Abtrennung der Katalysatorphase kann bei Temperaturen von -15°C bis 40°C, bevorzugt -10 bis 30°C, besonders bevorzugt -5 bis 20°C erfolgen. Die obere, Carbonsäurechlorid-haltige Phase wird im folgenden als Carbonsäurechlorid-Rohlösung bezeichnet. Zur Abtrennung der Katalysator-Phase können auch geeigenete Filter, wie beispielsweise Koaleszierfilter bekannter Bauart, eingesetzt werden.

Das erfindungsgemäße Verfahren schließt nicht aus, daß auch Carbonsäuren anderen Ursprungs in geringem Umfang zugeführt werden können.

Bevorzugt werden die zur Reinigung eingesetzten Carbonsäurechloride gemäß Formel (III) zum überwiegenden Teil aus der Umsetzung der entsprechenden Carbonsäuren mit Phosgen in Gegenwart des beschriebenen Katalysator-Addukts gewonnen.

In einer allgemeinen Ausführungsform zur diskontinuierlichen Herstellung der Carbonsäurechlorid-Rohlösung durch Umsetzung der Carbonsäure mit Phosgen wird das Katalysator-Addukt, welches durch Einleiten von Phosgen in N,N-disubstituiertes Formamid gewonnen werden kann, in einem Rührkessel vorgelegt und mit der Carbonsäure versetzt. Nach Einstellung der gewünschten Reaktionsbedingungen Temperatur und gegebenenfalls Druck wird die für die Reaktion erforderliche Menge gasförmigen oder flüssigen Phosgens unter Rühren innerhalb der gewünschten Zeit kontinuierlich zugeführt. Nach Beendigung der Reaktion wird der Rührkesselinhalt zur Phasentrennung in ein Trenngefäß überführt. Der Rührkessel steht somit einem weiteren Reaktionsansatz zur Verfügung. Nach etwa 1 bis 2 Stunden haben sich die beiden Phasen deutlich voneinander getrennt. Die untere Phase, welche in der Regel die katalysatorhaltige Phase ist, wird abgetrennt und die Carbonsäurechlorid-Phase, welche als Carbonsäurechlorid-Rohlösung bezeichnet wird, isoliert.

In einer allgemeinen Ausführungsform zur diskontinuierlichen Reinigung wird die Carbonsäurechlorid-Rohlösung in einen Rührkessel überführt, mit dem Carbonsäureamid versetzt und unter Rühren die gewünschte Menge Halogenwasserstoff eingeleitet. Dabei bildet sich eine zweite Phase, welche überwiegend aus dem Carbonsäureamid-hydrohalogenid besteht. Alternativ kann auch separat hergestelltes Carbonsäureamid-hydrohalogenid zugegeben werden. In dieser zweiten, Carbonsäureamid-hydrohalogenid-haltigen Phasen lösen sich unter Rühren die unerwünschten, farbgebenden Nebenkomponenten des Carbonsäurechlorids. Durch Abschalten des Rührers oder Überführung in ein weiteres Trenngefäß werden die beiden Phasen getrennt. Die Carbonsäurechlorid-haltige Phase kann nun, falls erforderlich, einer weiteren Reinigung, beispielsweise einer erneuten Extraktion mit Carbonsäureamid-hydrohalogenid, oder einer Entfernung gelösten Halogenwasserstoffs, beispielsweise durch Strippen mit Inertgas, wie z.B. Stickstoff oder Argon oder durch Evakuierung, unterzogen werden. Die Carbonsäureamidhydrohalogenid-haltige Phase kann gegebenenfalls erneut zur Extraktion eingesetzt werden. Falls ein N,N-disubstituiertes Formamid eingesetzt wird, welches mit der Katalysatorvorstufe identisch ist, so kann auch eine Beladung mit Phosgen und anschließendem Einsatz als Katalysator-Addukt in der Carbonsäurechlorid-Synthese erfolgen. Gleichzeitig ist auch eine partielle Ausschleusung zur Entfernung der angereicherten, farbgebenden Verunreinigungen möglich. Die ausgeschleuste Carbonsäureamid-hydrohalogenid-haltige Phase kann weiterhin auch einer Entsorgung oder destillativen Reinigung zur Abtrennung der Verunreinigungen zugeführt werden.

In einer weiteren allgemeinen Ausführungsform zur kontinuierlichen Herstellung der Carbonsäurechlorid-Rohlösung durch Umsetzung der Carbonsäure mit Phosgen werden in einem Rührkessel die Carbonsäure, rückgeführtes Katalysator-Addukt und gasförmiges oder flüssiges Phosgen unter den gewünschten Reaktionsbedingungen unter Rühren kontinuierlich zugefahren. Eine der zugeführten Menge entsprechende Menge wird kontinuierlich aus dem Rührkessel entnommen und einem Trenngefäß zugeführt. Aus diesem wird die katalysatorhaltige Phase, welche sich in der Regel unten befindet, kontinuierlich abgetrennt und erneut dem Rührkessel zugeführt. Zur Entfernung von Verunreinigungen ist es vorteilhaft, einen kleinen Teil zwischen 1 und 10 Gew.-% auszuschleusen und durch eine frische Katalysatorvorstufe zu ersetzen. Die Carbonsäurechlorid-Rohlösung wird ebenfalls kontinuierlich dem Trenngefäß entnommen.

In einer weiteren allgemeinen Ausführungsform zur kontinuierlichen Reinigung wird die Carbonsäurechlorid-Rohlösung zur Extraktion einer Rührkesselkaskade, mit z.B. 2 Rührkesseln zugeführt. Parallel zur Zufuhr der Carbonsäurechlorid-Rohlösung wird in den ersten Rührkessel rückgeführtes Carbonsäureamid-hydrohalogenid zugegeben. Zum Ersatz ausgetragenen Halogenwasserstoffs wird in den ersten Rührkessel gasförmiger Halogenwasserstoff eingeleitet. Nach Durchlaufen der einzelnen Kaskadenstufen gelangt der Ablauf des letzten Rührkessels in ein Trenngefäß, wo-sich beide Phasen voneinander trennen. Die Carbonsäurechlorid-haltige Phase wird kontinuierlich entnommen und wie unter der diskontinuierlichen Variante beschrieben weiterverarbeitet. Die Carbonsäureamid-hydrohalogenid-haltige Phase wird ebenfalls kontinuierlich entnommen und dem ersten Rührkessel wieder zugeführt. Zur Entfernung der extrahierten Verunreinigungen ist es vorteilhaft, einen Teil zwischen 1 und 20 Gew.-% auszuschleusen und durch frisches Carbonsäureamid oder dessen Hydrohalogenid zu ersetzen.

Wesentlich bei der oben beschriebenen erfindungsgemäßen Behandlung der Carbonsäurechlorid-Rohlösung ist dabei der überraschende Effekt, daß sich gerade die farbgebenden Komponenten in der Carbonsäureamid-hydrohalogenid-haltigen Phase erheblich besser lösen als in der Carbonsäurechlorid-haltigen Phase.

Das erfindungsgemäße Verfahren führt bereits durch eine einmalige Extraktion zu einer wesentlichen Verringerung der Farbzahl, so daß die derart gereinigten Carbonsäurechloride in der Regel ohne Destillation oder Adsorptivbehandlung für Folgereaktionen eingesetzt werden können. Das erfindungsgemäße Verfahren läßt sich sehr effektiv und wirtschaftlich durchführen. Durch Umgehung der nach Stand der Technik üblichen Destillation werden sowohl Investitions- und Energiekosten eingespart als auch in der Regel eine höhere Ausbeute an gereinigtem Carbonsäurechlorid erreicht. Für destillationsempfindliche Carbonsäurechloride eröffnet das erfindungsgemäße Verfahren die Möglichkeit einer wirtschaftlichen Synthese im technischen Maßstab.

### Beispiele

### Synthese 1: Herstellung von N,N-Dimethylformamid-hydrochlorid

Die Synthese von N,N-Dimethylformamid-hydrochlorid ist in I.S. Kislina et al., Russ. Chem. Bl., EN, 43(9), 1994, 1505-1507 beschrieben. 365,5 g (5,0 mol) N,N-Dimethylformamid (DMF) wurden in eine Rührapparatur vorgelegt und auf 45°C erwärmt. Anschließend wurde unter Rühren gasförmiges HCl bis zum Einsetzen von Abgasmenge eingeleitet. Es wurde eine klare, farblose Flüssigkeit erhalten, die nach der Elementaranalyse einer Zusammensetzung von DMF*2HCl entsprach.

### Verfahren 1: Diskontinuierliche Herstellung der Carbonsäurechlorid-Rohlösung über Phosgen

Zur Herstellung der Carbonsäurechlorid-Rohlösungen nach diskontinuierlichem Verfahren wurden jeweils 2 bis 5 mol der entsprechenden Carbonsäuren in eine Rührapparatur vorgelegt und mit 10 bis 50 mol-% bezogen auf die eingesetzte Carbonsäure mit N,N-Dimethylformamid versetzt. Die Reaktionslösung wurde unter Rühren auf eine Temperatur von 25 bis 45°C gebracht und unter Atmosphärendruck gasförmiges Phosgen eingeleitet. Nachdem die, bezogen auf die vorgelegte Carbonsäuremenge, stöchiometrische Menge an Phosgen zuzüglich des gewünschten Überschusses eingeleitet wurde, wurde die weitere Zufuhr gestoppt und das System bei ausgeschaltetem Rührer 2 Stunden stehen gelassen. Nach Trennung der beiden Phasen wurde die Carbonsäurechlorid-Rohlösung als obere Phase isoliert und die Farbzahl nach APHA bestimmt.

### Verfahren 2: Kontinuierliche Herstellung der Carbonsäurechlorid-Rohlösung über Phosgen

Zur Herstellung der Carbonsäurechlorid-Rohlösungen nach kontinuierlichem Verfahren wurden jeweils 0,75 mol/h der entsprechenden Carbonsäuren, 30 g/h rückgeführtes Katalysator-Addukt sowie 0,75 bis 0,80 mol/h gasförmiges Phosgen bei einer Temperatur von 45°C und einem Druck von 1 bar abs in eine Rührapparatur eingeleitet. Durch die vorhandene Standregelung wurde die entsprechende Menge an Reaktionsgemisch abgeführt und in ein Trenngefäß geleitet. Die untere, katalysatorhaltige Phase wurde rückgeführt. Die obere, carbonsäurechloridhaltige Phase wurde als Carbonsäurechlorid-Rohlösung isoliert und die Farbzahl nach APHA bestimmt.

### Beispiel 1: Reinigung von Laurinsäurechlorid

Aus Laurinsäure und Phosgen wurde nach diskontinuierlichem Verfahren 1 eine Laurinsäurechlorid-Rohlösung mit einer Farbzahl von 268 APHA hergestellt. 200 g dieses Produkts wurden mit 50 g DMF-Hydrochlorid aus Synthese 1 in einer Rührapparatur intensiv gerührt und anschließend die Phasen getrennt. Die Laurinsäurechlorid-Phase wurde mit Stickstoff HCl-frei gestrippt. Die Farbzahl betrug nur noch 48 APHA.

### Beispiel 2: Reinigung von Kokosfettsäurechlorid

Aus Kokosfettsäure (Handelsname HK 8-18, Fa. Henkel), welche im wesentlichen aus Laurinsäure und Myristinsäure besteht, und Phosgen wurde nach diskontinuierlichem Verfahren 1 eine Kokosfettsäurechlorid-Rohlösung mit einer Farbzahl von 399 APHA hergestellt. 200 g dieses Produkts wurden mit 50 g DMF-Hydrochlorid aus Synthese 1 in einer Rührapparatur intensiv gerührt und anschließend die Phasen getrennt. Die Kokosfettsäurechlorid-Phase wurde mit Stickstoff HCl-frei gestrippt. Die Farbzahl betrug nur noch 64 APHA.

### Beispiel 3: Reinigung von Pelargonsäurechlorid (Nonansäurechlorid)

Aus Pelargonsäure (Nonansäure) und Phosgen wurde nach kontinuierlichem Verfahren 2 eine Pelargonsäurechlorid-Rohlösung mit einer Farbzahl von 301 APHA hergestellt. 90 g dieses Produkts wurden mit 10 g DMF-Hydrochlorid aus Synthese 1 in einer Rührapparatur intensiv gerührt und anschließend die Phasen getrennt. Die Pelargonsäurechlorid-Phase wurde mit Stickstoff HCl-frei gestrippt. Die Farbzahl betrug nur noch 55 APHA. Eine Wiederholung der Extraktion mit weiteren 10 g DMF-Hydrochlorid aus Synthese 1 führte zu einer Farbzahl von 36 APHA. Nach einer dritten, analog durchgeführten Extraktion betrug die Farbzahl nur noch 30 APHA.

### Beispiel 4: Reinigung von Pelargonsäurechlorid (Nonansäurechlorid)

Aus Pelargonsäure (Nonansäure). und Phosgen wurde nach diskontinuierlichem Verfahren 1 eine Pelargonsäurechlorid-Rohlösung mit einer Farbzahl von 118 APHA hergestellt. 90 g dieses Produkts wurden mit 10 g DMF-Hydrochlorid aus Synthese 1 in einer Rührapparatur intensiv gerührt und anschließend die Phasen getrennt. Die Pelargonsäurechlorid-Phase wurde mit Stickstoff HCl-frei gestrippt. Die Farbzahl betrug nur noch 50 APHA. Eine Wiederholung der Extraktion mit weiteren 10 g DMF-Hydrochlorid aus Synthese 1 führte zu einer Farbzahl von 48 APHA. Nach einer dritten, analog durchgeführten Extraktion betrug die Farbzahl 45 APHA.

### Beispiel 5: Reinigung von Pivalinsäurechlorid

Aus Pivalinsäure und Phosgen wurde nach kontinuierlichem Verfahren 2 eine Pivalinsäurechlorid-Rohlösung mit einer Farbzahl von 361 APHA hergestellt. 200 g dieses Produkts wurden mit 50 g DMF-Hydrochlorid aus Synthese 1 in einer Rührapparatur intensiv gerührt und anschließend die Phasen getrennt. Die Pivalinsäurechlorid-Phase wurde mit Stickstoff HCl-frei gestrippt. Die Farbzahl betrug nur noch 35 APHA.

### Beispiel 6: Reinigung von Pivalinsäurechlorid (Wiederholung)

Aus Pivalinsäure und Phosgen wurde nach kontinuierlichem Verfahren 2 eine Pivalinsäurechlorid-Rohlösung mit einer Farbzahl von 409 APHA hergestellt. 200 g dieses Produkts wurden mit 50 g DMF-Hydrochlorid aus Synthese 1 in einer Rührapparatur intensiv gerührt und anschließend die Phasen getrennt. Die Pivalinsäurechlorid-Phase wurde mit Stickstoff HCl-frei gestrippt. Die Farbzahl betrug nun 83 APHA.

Da im Vergleich zu Beispiel 5 im vorliegenden Beispiel von einer Rohlösung mit einer höheren Farbzahl ausgegangen wurde, wurde auch eine gereinigte Lösung mit höherer Farbzahl erhalten. Die Abreicherung der farbigen Komponenten ist in beiden Beispielen jedoch vergleichbar.

### Beispiel 7: Reinigung von Ölsäuresäurechlorid

Aus Ölsäure und Phosgen wurde nach kontinuierlichem Verfahren 2 eine Ölsäurechlorid-Rohlösung mit einer Iodfarbzahl von 38 hergestellt. 200 g dieses Produkts wurden mit 50 g DMF-Hydrochlorid aus Synthese 1 in einer Rührapparatur intensiv gerührt und anschließend die Phasen getrennt. Die Ölsäurechlorid-Phase wurde mit Stickstoff HCl-frei gestrippt. Die Iodfarbzahl betrug nur noch 26.

### Beispiel 8: Reinigung von Palmitinsäurechlorid

Aus Palmitinsäure und Phosgen wurde nach diskontinuierlichem Verfahren 1 eine Palmitinsäurechlorid-Rohlösung mit einer Farbzahl von 202 APHA hergestellt. 20 ml dieses Produkts wurden mit 5 ml DMF-Hydrochlorid aus Synthese 1 in einer Rührapparatur intensiv gerührt und anschließend die Phasen getrennt. Die Palmitinsäurechlorid-Phase wurde mit Stickstoff HCl-frei gestrippt. Die Farbzahl betrug nur noch 82 APHA.

### Beispiel 9: Reinigung von Pelargonsäurechlorid (Nonansäurechlorid)

158 g (1,0 mol) Pelargonsäure wurden mit 0,4 g (0,005 mol) N,N-Dimethylformamid versetzt und auf 50°C erwärmt. Bei 50°C wurden innerhalb von 45 Minuten insgesamt 125 g (1,05 mol) Thionylchlorid zugetropft. Nach einer Nachreaktionszeit von 30 Minuten bei 50°C wurde bei 50°C eine Stunde lang Stickstoff durchgeleitet und Schwefeldioxid, Chlorwasserstoffgas und nicht-umgesetztes Thionylchlorid ausgestrippt. Der hellgelbe Austrag hatte eine Farbzahl von 113 APHA und enthielt nach einer GC-Analyse 99,5 Flächen-% Pelargonsäurechlorid.

Vom Austrag wurden 20 ml mit 5 ml DMF-Hydrochlorid aus Synthese 1 in einer Rührapparatur intensiv gerührt und anschließend die Phasen getrennt. Die Pelargonsäurechlorid-Phase wurde mit Stickstoff HCl-frei gestrippt. Die Farbzahl betrug nur noch 37 APHA.

Die Beispiele zeigen für Carbonsäurechloride aus beiden Syntheserouten, via Phosgen und via Thionylchlorid, daß unabhängig von der Art der Carbonsäure, d.h. unabhängig davon, ob es sich um gesättigte oder ungesättigte, um geradkettige oder verzweigte Carbonsäuren handelt, die Farbzahl durch die erfindungsgemäße Behandlung (Extraktion) deutlich verringert werden kann. Eine wiederholte Extraktion führt dabei zu einer weiteren Verringerung der Farbzahl. Die in den Beispielen erhaltenen Carbonsäurechloride können ohne weitere Reinigungsschritte in Folgesynthesen eingesetzt werden.

## Patentansprüche

1. Verfahren zur Reinigung von Carbonsäurechloriden, die durch Umsetzung von Carbonsäuren mit Phosgen oder Thionylchlorid in Gegenwart eines Katalysator-Addukts hergestellt und von der das Katalysator-Addukt enthaltenden Phase abgetrennt wurden, **dadurch gekennzeichnet, daß** man die Carbonsäurechloride mit einem Hydrohalogenid von Carbonsäureamiden der allgemeinen Formel (I) 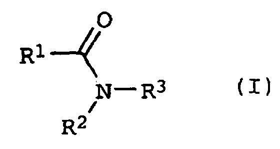 in der R¹ für Wasserstoff oder C₁- bis C₃-Alkyl steht; R² und R³ unabhängig voneinander C₁- bis C₄-Alkyl oder R² und R³ gemeinsam eine C₄- oder C₅-Alkylenkette bedeuten, behandelt, wobei die gegenseitige Löslichkeit der Carbonsäurechloride und der Hydrohalogenide der Carbonsäureamide (I) gering ist, und das so gereinigte Carbonsäurechlorid durch Abtrennung von der Carbonsäureamid-hydrohalogenid-Phase isoliert.

2. Verfahren nach Anspruch 1, **dadurch gekennzeichnet, daß** man zur Behandlung der Carbonsäurechloride eine Carbonsäureamidhydrohalogenid-Menge von 1 bis 80 Gew.-%, bezogen auf die eingesetzte Carbonsäurechlorid-Menge, verwendet.

3. Verfahren nach den Ansprüchen 1 bis 2, **dadurch gekennzeichnet, daß** man als Carbonsäureamid-hydrohalogenid N,N-Dimethylformamid-hydrochlorid einsetzt.

4. Verfahren nach den Ansprüchen 1 bis 3, **dadurch gekennzeichnet, daß** man die Behandlung mit dem Carbonsäureamid-hydrohalogenid bei einer Temperatur von -15 bis 80°C und einem Druck von 0,5 bis 5,0 bar abs durchführt.

5. Verfahren nach den Ansprüchen 1 bis 4, **dadurch gekennzeichnet, daß** man als Katalysatorvorstufe für das zu bildende Katalysator-Addukt ein N,N-disubstituiertes Formamid der allgemeinen Formel (II) 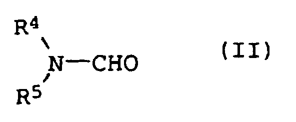 in der R⁴ und R⁵ unabhängig voneinander C₁- bis C₄-Alkyl oder R⁴ und R⁵ gemeinsam eine C₄- oder C₅-Alkylenkette bedeuten, einsetzt.

6. Verfahren nach den Ansprüchen 1 bis 5, **dadurch gekennzeichnet, daß** man als Katalysatorvorstufe gemäß der allgemeinen Formel (II) N,N-Dimethylformamid einsetzt.

7. Verfahren nach den Ansprüchen 3 bis 6, **dadurch gekennzeichnet, daß** man das N,N-Dimethylformamid-hydrochlorid nach der Nutzung als Behandlungsmittel als Katalysatorvorstufe in der Carbonsäurechlorid-Synthese einsetzt.

8. Verfahren nach den Ansprüchen 1 bis 7, **dadurch gekennzeichnet, daß** die eingesetzten Carbonsäurechloride zum überwiegenden Teil aus der Umsetzung von Carbonsäuren mit Phosgen in Gegenwart eines Katalysator-Addukts stammen.

9. Verfahren nach den Ansprüchen 1 bis 8, **dadurch gekennzeichnet, daß** man als zu reinigende Carbonsäurechloride Essigsäurechlorid, Propionsäurechlorid, Buttersäurechlorid, Valeriansäurechlorid, Isovaleriansäurechlorid, Pivalinsäurechlorid, Capronsäurechlorid, 2-Ethylbuttersäurechlorid, Önanthsäurechlorid, Caprylsäurechlorid, 2-Ethylhexansäurechlorid, Pelargonsäurechlorid, Isononansäurechlorid, Caprinsäurechlorid, Neodecansäurechlorid, Laurinsäurechlorid, Myristinsäurechlorid, Palmitinsäurechlorid, Stearinsäurechlorid, Ölsäurechlorid, Linolsäurechlorid, Linolensäurechlorid, Arachidinsäurechlorid und Behensäurechlorid sowie deren Mischungen einsetzt.

## Claims

1. A process for the purification of carbonyl chlorides which have been prepared by reacting carboxylic acids with phosgene or thionyl chloride in the presence of a catalyst adduct and have been separated off from the phase containing the catalyst adduct, which comprises treating the carbonyl chlorides with a hydrohalide of carboxamides of the formula (I) 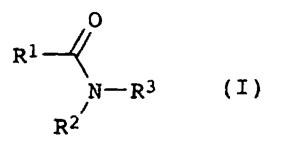 in which R¹ is hydrogen or a C₁- to C₃-alkyl; R² and R³ independently of one another are C₁- to C₄-alkyl, or R² and R³ together are a C₄- or C₅-alkylene chain, the mutual solubility of the carbonyl chlorides and the hydrohalides of the carboxamides (I) being low, and isolating the carbonyl chloride purified in this way by separation from the carboxamide hydrohalide phase.

2. A process as claimed in claim 1, wherein, for the treatment of the carbonyl chlorides, an amount of carboxamide hydrohalide of from 1 to 80% by weight, based on the amount of carbonyl chloride employed, is used.

3. A process as claimed in claims 1 to 2, wherein the carboxamide hydrohalide used is N,N-dimethylformamide hydrochloride.

4. A process as claimed in claims 1 to 3, wherein the treatment with the carboxamide hydrohalide is carried out at a temperature of from -15 to 80°C and a pressure of from 0.5 to 5.0 bar abs.

5. A process as claimed in claims 1 to 4, wherein, as catalyst precursor for the catalyst adduct to be formed, an N,N-disubstituted formamide of the formula (II) is used 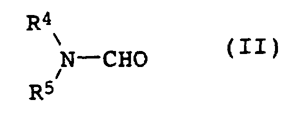 in which R⁴ and R⁵ independently of one another are C₁- to C₄-alkyl, or R⁴ and R⁵ together are a C₄- or C₅-alkylene chain.

6. A process as claimed in claims 1 to 5, wherein the catalyst precursor according to the formula (II) used is N,N-dimethylformamide.

7. A process as claimed in claims 3 to 6, wherein the N,N-dimethylformamide hydrochloride, after it has been used as treatment agent, is used as catalyst precursor in the carbonyl chloride synthesis.

8. A process as claimed in claims 1 to 7, wherein most of the carbonyl chlorides used originate from the reaction of carboxylic acids with phosgene in the presence of a catalyst adduct.

9. A process as claimed in claims 1 to 8, wherein the carbonyl chlorides to be purified are acetyl chloride, propionyl chloride, butyryl chloride, valeryl chloride, isovaleryl chloride, pivaloyl chloride, caproyl chloride, 2-ethylbutyryl chloride, enanthyl chloride, capryloyl chloride, 2-ethylhexanoyl chloride, pelargonoyl chloride, isononanoyl chloride, capryl chloride, neodecanoyl chloride, lauroyl chloride, myristoyl chloride, palmitoyl chloride, stearoyl chloride, oleoyl chloride, linoleoyl chloride, linolenoyl chloride, arachidoyl chloride and behenoyl chloride, and mixtures thereof.

## Revendications

1. Procédé de purification de chlorures d'acides carboxyliques, préparés par réaction d'acides carboxyliques avec du phosgène ou du chlorure de thionyle en présence d'un produit d'addition de catalyseur et séparés de la phase contenant le produit d'addition de catalyseur, **caractérisé en ce que** l'on traite les chlorures d'acides carboxyliques par un hydrohalogénure d'amide carboxylique de la formule générale (I) 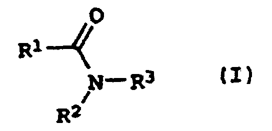 dans laquelle R¹ représente de l'hydrogène ou un radical alkyle en C₁ à C₃, R² et R³ représentent indépendamment l'un de l'autre des radicaux alkyle en C₁ à C₄, ou bien R² et R³ représentent ensemble une chaîne alkylène en C₄ ou C₅, la solubilité mutuelle des chlorures d'acides carboxyliques et de l'hydrohalogénure d'amide carboxylique (I) étant faible, et **en ce que** le chlorure d'acide carboxylique ainsi purifié est isolé par séparation de la phase d'hydrohalogénure d'amide carboxylique.

2. Procédé selon la revendication 1, **caractérisé en ce que**, pour le traitement des chlorures d'acides carboxyliques, on utilise une quantité d'hydrohalogénure d'amide carboxylique de 1 à 80% en poids, par rapport à la quantité de chlorure d'acide carboxylique mise en oeuvre.

3. Procédé selon les revendications 1 à 2, **caractérisé en ce que** l'on met en oeuvre comme hydrohalogénure d'amide carboxylique du chlorhydrate de N,N-diméthylformamide.

4. Procédé selon les revendications 1 à 3, **caractérisé en ce que** l'on entreprend le traitement par l'hydrohalogénure d'amide carboxylique à une température de -15 à 80°C et une pression de 0,5 à 5,0 bar absolus.

5. Procédé selon les revendications 1 à 4, **caractérisé en ce que** l'on met en oeuvre comme précurseur de catalyseur pour le produit d'addition de catalyseur à former un formamide N,N-disubstitué de la formule générale (II) 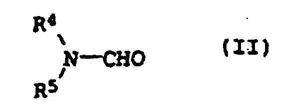 dans laquelle R⁴ et R⁵ représentent indépendamment l'un de l'autre un radical alkyle en C₁ à C₄ ou bien R⁴ et R⁵ représentent ensemble une chaîne alkylène en C₄ ou C₅.

6. Procédé selon les revendications 1 à 5, **caractérisé en ce que** l'on met en oeuvre comme précurseur de catalyseur selon la formule générale (II) du N,N-diméthylformamide.

7. Procédé selon les revendications 3 à 6, **caractérisé en ce que** l'on met en oeuvre le chlorhydrate de N,N-diméthylformamide après l'utilisation comme agent de traitement en tant que précurseur de catalyseur dans la synthèse de chlorure d'acide carboxylique.

8. Procédé selon les revendications 1 à 7, **caractérisé en ce que** les chlorures d'acides carboxyliques mis en oeuvre proviennent en majeure partie de la réaction d'acides carboxyliques avec du phosgène en présence d'un produit d'addition de catalyseur.

9. Procédé selon les revendications 1 à 8, **caractérisé en ce que** l'on met en oeuvre comme chlorures d'acides carboxyliques à purifier du chlorure d'acide acétique, du chlorure d'acide propionique, du chlorure d'acide butyrique, du chlorure d'acide valérianique, du chlorure d'acide isovalérianique, du chlorure d'acide pivalique, du chlorure d'acide caproïque, du chlorure d'acide 2-éthylbutyrique, du chlorure d'acide oenanthique, du chlorure d'acide caprylique, du chlorure d'acide 2-éthylhexanoïque, du chlorure d'acide pélargonique, du chlorure d'acide isononanoïque, du chlorure d'acide caprique, du chlorure d'acide néodécanoïque, du chlorure d'acide laurique, du chlorure d'acide myristique, du chlorure d'acide palmitique, du chlorure d'acide stéarique, du chlorure d'acide oléique, du chlorure d'acide linoléique, du chlorure d'acide linolénique, du chlorure d'acide arachidique et du chlorure d'acide béhénique, ou leurs mélanges.
